(19) Europäisches Patentamt
European Patent Office
Office européen des brevets

(11) Veröffentlichungsnummer: **0 178 436**
**B1**

(12) **EUROPÄISCHE PATENTSCHRIFT**

(45) Veröffentlichungstag der Patentschrift: 24.10.90

(51) Int. Cl.⁵: **A 61 K 31/00, A 61 K 47/00**

(21) Anmeldenummer: 85111013.0

(22) Anmeldetag: 31.08.85

(54) Ibuprofen enthaltende Weichgelatinekapseln und Verfahren zu ihrer Herstellung.

(30) Priorität: 13.10.84 DE 3437599

(43) Veröffentlichungstag der Anmeldung:
23.04.86 Patentblatt 86/17

(45) Bekanntmachung des Hinweises auf die
Patenterteilung:
24.10.90 Patentblatt 90/43

(84) Benannte Vertragsstaaten:
AT BE CH DE FR GB IT LI LU NL SE

(56) Entgegenhaltungen:
EP-A-0 070 714
EP-A-0 087 062
DE-A-2 631 282

P.H. LIST et al.: "HAGERS HANDBUCH DER
PHARMAZEUTISCHEN PRAXIS", vierte
Neuausgabe, Band 7, Teil B: Hilfsstoffe, 1977,
Seiten 407-409, Springer Verlag, Berlin,
Heidelberg, New York.

(73) Patentinhaber: Dolorgiet GmbH & Co. KG
Otto-von-Guericke-Str. 1
D-5205 St. Augustin 3 (DE)

(72) Erfinder: Löhner, Manfred
Blücherstrasse 47
D-5300 Bonn 1 (DE)
Erfinder: Posselt, Klaus, Dr.
Rodderbergstrasse 62
D-5300 Bonn 2 (DE)

(74) Vertreter: Werner, Hans-Karsten, Dr. et al
Deichmannhaus am Hauptbahnhof
D-5000 Köln 1 (DE)

(56) Entgegenhaltungen:
CHEMICAL ABSTRACTS, Band 101, Nr. 18, 29.
Oktober 1984, Seite 356, Nr. 157696h,
Columbus, Ohio, US; & JP - A - 59 122 425
(KAKEN PHARMACEUTICAL CO., LTD.) 14-07-
1984

Römpps Chemie Lexikon, 8 Auflage, Stuttgart
1987 S. 3260 und 3276

**Beschreibung**

Gegenstand der vorliegenden Erfindung sind Ibuprofen enthaltende Weichgelatinekapseln sowie Verfahren zu ihrer Herstellung.

Die Verbindung Ibuprofen, 2-(4-Isobutyl-phenyl)-propionsäure, ist z.B. aus Matindale, The Extra Pharmacopoeia, 28th edition, 1982, S, 256 als Arzneistoff bekannt, der antiinflammatorische und analgetische Eigenschaften besitzt. Er wird eingesetzt zur Behandlung von rheumatoider Arthritis oder anderen entzündlichen Gelenkerkrankungen, Weichteilrheumatismus und Gicht. Wegen seiner analgetischen Eigenschaften findet Ibuprofen aucy eine breite Anwendung als Schmerzmittel, z.B. bei Kopf- und Zahnschmerzen sowie Menstruationsbeschwerden.

Von einem Arzneimittel, das sich zur Bekämpfung akuter Schmerzen eignet, wird ein rascher Wirkungseintritt geforder, der wiederum nur durch eine rasche Freisetzung und gute Bioverfügbarkeit des Wirkstoffes erreicht wird. Bislang ist Ibuprofen zur enteralen Verabreichung nur in fester Form als Dragées oder überzogene Tabletten im Handel. Aus den DE-OSen 28 32 380 und 31 24 014 sind flüssige Suspensionen bekannt, in denen Ibuprofen als Aluminiumsalz vorliegt. Hinsichtlich der Zusammensetzung und wegen des Volumens, das eingenommen werden muß, um eine wirksame Dosis an Ibuprofen zu erreichen, eignen sich diese Suspensionen nicht zur Einarbeitung in eine Kapsel. Die DE-OS 33 40 347 und EP-OS 0 070 714 beschrieben neben anderen Darreichungsformen auch Weichgelatinkapseln, in denen Ibuprofen suspendiert in einem Träger enthalten ist. Bei allen genannten Darreichungsformen müssen, damit eine gute Verfügbarkeit des Ibuprofens gewährleistet ist, hohe Anforderungen an die physikalischen Eigenschaften des Wirkstoffes, wie Teilchengröße und spezifische Oberfläche gestellt werden. Vor allem bei den handelsüblichen Formen (Tabletten, Dragées) müssen die Herstellungsbedingungen streng eingehalten werden, da bereits geringe Veränderungen des Produktionsablaufs, wie Mischen, Preßdruck oder Maschinentyp, die physikalischen Eigenschaften der Wirkstoffteilchen beeinflussen und die Bioverfügbarkeit beeinträchtigen.

Die Erfindung hat sich zur Aufgabe gestellt, ein gut einnehmbares Arzneimittel zur Verfügung zu stellen, welches eine wirksame Menge Ibuprofen in einem Träger enthält, das einfach herstellbar ist und rasch eine hohe Wirksamkeit entfaltet. Ibuprofen ist zwar in einigen physiologisch verträglichen Lösungsmitteln löslich, fällt aber bei Zugabe von wenig Wasser oder beim Einbringen der Lösung in wässrige Medien, wie z.B. künstlichen Magensaft, sofort aus. Gelangt also eine solche Lösung bei oraler Einnahme in den Magen, so bewirkt der wässrige Mageninhalt eine Ausfällung des Ibuprofens, das damit einer schnellen Resoprtion entzogen wird.

Es wurde jetzt gefunden, daß Ibuprofen gelöst in Polyoxyethylenpolyoxypropylen-Diol, das zugleich als Tensid fungieren kann, oder in einem Gemisch aus Polyoxyethylen-polyoxypropylen-Diol oder Polyethylenglykol oder Polypropylenglykol und einem physiologisch verträglichen Tensid bei Temperaturen von 45 bis 65°C gut gelöst werden kann und beim Abkühlen auf Raumtemperatur gelöst bleibt. Es wurde weiterhin gefunden, daß überraschenderweise beim Einbringen dieser Lösungen in wässrigen Medien, insbesondere künstlichen Magensaft keine Ausfällung erfolgt, so daß das Ibuprofen aus dieser Lösung heraus rasch und vollständig resorbiert werden kann. Diese Lösungen können obendrein in an sich bekannter Weise in Weichgelatinekapseln eingearbeitet werden. Diese Darreichungsform weist gegenüber allen bisher bekannten Darreichungsformen des Ibuprofens den Vorteil auf, daß nach der Einnahme der Wirkstoff rasch resorbiert wird. Dabei ist es nicht notwendig, den Wirkstoff nach den Synthese durch aufwendige galenische Maßnahmen zu verarbeiten. Trotzdem erhält man zuverlässig reproduzierbar hohe Bioverfügbarkeiten und damit einen raschen und zuverlässigen Wirkungseintritt.

Die erfindungsgemäßen Effekte werden erzielt, wenn die Ibuprofen enthaltenden Weichgelatinekapseln eine Lösung von 15 bis 30 Gewichtsteilen Ibuprofen in 70 bis 85 Gewichtsteilen Polyoxyethylen-polyoxypropylen-Diol oder in einem Gemisch aus 30 bis 76 Gewichtsteilen Polyoxyethylen-polyoxypropylen-Diol oder Polyethylenglykol oder Polypropylenglyol und 7 bis 40 Gewichtsteilen eines physiologisch verträglichen Tensids enthalten. Zur leichteren Verarbeitbarkeit und Erhöhung der Löslichkeit können gewünschtenfalls bis zu 3 Gewichtsteile 1,2-Propylenglykol zugesetzt werden, ohne daß dadurch die Eigenschaften beim Zusammenbringen mit einem wässrigen Medium wie künstlichen Magensaft verschlechtert werden.

Ein weiterer Vorteil der erfindungsgemäßen Lösungen ist, daß hierin zusätzlich bis zu 40 Gewichtsteile Ibuprofen suspendiert werden können, so daß man Darreichungsformen enthält, die auch bei hoher Dosierung relativ klein dimensioniert und daher leicht einnehmbar sind. Diese Darreichungsform kommt insbesondere für die Behandlung rheumatischer Erkrankungen in Frage.

Als Lösungsmittel kommt erfindungsgemäß vor allem Polyoxyethylen-polyoxypropylen-Diol, das zugleich Tensid sein kann, in Frage. Dieses Diol sollte vorzugsweise relative Molmassen von 1400 bis 2000 aufweisen. Derartige Produkte sind beispielsweise unter dem Warenzeichen Pluronic[R] im Handel erhältlich. Sie sind physiologisch verträglich und deshalb als Hilfsmittel zur Herstellung von Weichgelatinekapseln geeignet.

Als weiteres Lösungsmittel kommen Gemische aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids in Frage. Verwendung finden vor allem Polyethylenglykol oder Polypropylenglykol, deren relative Molmasse vorzugsweise im Bereich zwischen 300 und 630 liegen sollte.

2

Auch diese Produkte sind im Handel erhältlich, physiologisch unbedenklich und daher für den erfungsgemäßen Zweck einsetzbar. Bie Verwendung von Polyethylenglykol oder Polypropylenglykol ist es notwendig 30—76 Gewichtsteilen des Glykols zusätzlich 7 bis 40 Gewichtsteile eines physiologisch verträglichen Tensides zuzusetzen, um die Wiederausfällung des Wirkstoffes durch wässrige Medien wie künstlichen Magensaft zu verhindern. Als Tenside kommen insbesondere Polyoxyethylenglycerol-tri-hydroxystearat, Polyoxyethylen-($C_{12-18}$)-fettalkoholether, Polyoxyethylen-stearat, Polyoxyethylen-sorbitan-mono-($C_{12-18}$)-fettsäureester oder auch Polyoxyethylen-polyoxypropylen-Diole in Frage. Die Ethylenoxydeinheiten des Polyoxyethylenglycerol-tri-hydroxystearats sollen vorzugsweise zwischen 35 und 65 liegen und die des Polyoxyethylen-($C_{12-18}$)-fettalkoholethers zwischen 15 und 25. Die Ethylenoxydeinheiten des Polyoxyethylen-stearats sollen vorzugsweise 15 bis 45 betragen und die des Polyoxyethylen-sorbitan-mono-($C_{12-18}$)-fettsäureesters 15 bis 25.

Um Ibuprofen in den erfindungsgemäß verwendeten Lösungsmitteln zu lösen, werden diese auf 45 bis 65°C erwärmt, da nur bei diesen Temperaturen eine ausreichend rasche Lösung erfolgt. Beim Abkühlen auf Raumtemperatur erfolgt keine Wiederausfällung. Sofern gewünschtenfalls weitere Mengen Ibuprofen in die Weichgelatinekapsel eingebracht werden sollen, kann dieses in der Lösung bei Raumtemperatur suspendiert werden. Es ist ohne weiteres möglich, bis zu 40 Gewichtsteile Ibuprofen zusätzlich in diesen Lösungen bei Raumtemperatur zu suspendieren und dann in Weichgelatinekapseln einzuarbeiten. Sofern Gemische der genannten Lösungsmittel und einem Tensid eingesetzt werden, werden diese zuvor bei Temperaturen bis zu 80°C homogen miteinander vermischt und dann auf ca. 50 bis 60°C abgekühlt. Bei diesen Temperaturen wird dann das Ibuprofen eingebracht und gelöst.

Gegenstand der vorliegenden Erfindung sind somit nicht nur die oben beschriebenen Weichgelatinekapseln sondern auch Verfahren zu ihrer Herstellung, dadurch gekennzeichnet, daß 15 bis 30 Gewichtsteile Ibuprofen bei Temperaturen zwischen 45 und 65°C in 70 bis 85 Gewichtsteilen Polyoxyethylen-polyoxypropylen-Diol oder in einem Gemisch aus 30 bis 76 Gewichtsteilen Polyoxyethylen-polyoxypropylen-Diol oder Polyethylen-oder Polypropylenglykol und 7 bis 40 Gewichtsteilen eines physiologisch verträglichen Tensids gelöst, auf Raumtemperatur abgekühlt und in an sich bekannter Weise in Weichgelatinkapseln eingearbeiten werden.

Schließlich betrifft die Erfindung die Verwendung von Lösungen enthaltend 15 bis 30 Gewichtsteile Ibuprofen in 70 bis 85 Gewichtsteilen Polyoxyethylen-Polyoxypropylen-Diol oder in einem Gemisch aus 30 bis 76 Gewichtsteilen Polyoxyethylen-polyoxypropylen-Diol oder Polyethylen- oder Polypropylenglykol und 7 bis 40 Gewichtsteilen eines physiologisch verträglichen Tensids zur Herstellung von Weichgelatinekapseln.

In den folgenden Beispielen sind die erfindungsgemäßen Weichgelatinekapseln sowie die Verfahren zu ihrer Herstellung näher erläutert.

Beispiel 1

Man stellt unter Rühren bei 70 bis 80°C eine Mischung aus 270,62 g Polyethylenglykol 400, 29,26 g Polyoxyethylen-(40)-glycerol-tri-hydroxystearat (Cremophor[R] RH 40, BASF) und 6,12 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0.880 g Lösung enthalten 200 mg Ibuprofen.

Beispiel 2

Man stellt unter Rühren bei 70 bis 80°C eine Mischung aus 264,50 g Polyethylenglykol 400 und 29,26 g Polyoxyethylen-(40)-glycerol-tri-hydroxystearat (Cremophor[R] RH 40, BASF) her, kühlt auf 50°C ab und rührt nacheinander portionsweise 90,00 g Ibuprofen und 12,24 g 1,2-Propylenglykol ein. Die Lösung wird auf Raumtemperatur abgekühlt und in Weichgelatinekapseln geeigneter Größe eingearbeitet. 0.880 g Lösung enthalten 200 mg Ibuprofen.

Beispiel 3

Man löst 90,00 g Ibuprofen unter Rühren bei 50 bis 60°C in 224,74 g Polyethylenglykol 400 und rührt dann nacheinander bei dieser Temperatur 24,86 g Polyoxyethylen-(40)-glycerol-tri-hydroxystearat (Cremophor[R] RH 40, BASF) und 10,40 g 1,2-Propylenglykol ein. Die Lösung wird auf Raumtemperatur abgekühlt und in Weichgelatinekapseln geeigneter Größe eingearbeitet. 0.778 g Lösung enthalten 200 mg Ibuprofen.

Beispiel 4

Man stellt unter Rühren bei 70 bis 80°C eine Mischung aus 270,62 g Polypropylenglykol 425, 29,26 g Polyoxyethylne-(40)-glycerol-tri-hydroxystearat (Cremophor[R] RH 40, BASF) und 6,12 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 5

Man stellt unter Rühren bbi 60 bis 70°C eine Mischung aus 270,70 g Polyethylenglykol 600, 29,30 g Polyoxyethylen-(40)-glycerol-tri-hydroxystearat (Cremophor^R RH 40, BASF) und 6,00 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 6

Man stellt unter Rühren bei 60°C eine Mischung aus 30,773 kg Polyoxyethylen-polyoxypropylen-Diol 1900 (Pluronic^R L 35, Wyandotte Chemicals Corp.), 3,325 kg Polyoxyethylen-(40)-glycerol-tri-hydroxystearat (Cremophor^R RH 40, BASF) und 0,675 kg 1,2-Propylenglykol her und rührt portionsweise 10,227 kg Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 7

Man stellt unter Rühren bei 60°C eine Mischung aus 391,24 g Polyoxyethylen-polyoxypropylen-Diol 1500 (Pluronic^R L 42, Wyandotte Chemicals Corp.), 39,18 g Polyoxyethylen-(40)-glycerol-tri-hydroxystearat (Cremophor^R RH 40, BASF) und 9,00 g 1,2-Propylenglykol her und rührt portionsweise 90,00 g Ibuprofen ein. Die Lösung wird auf Raumtemperatur abgekühlt und in Weichgelatinekapseln geeigneter Größe eingearbeitet. 0,882 g Lösung enthalten 150 mg Ibuprofen.

Beispiel 8

Man stellt unter Rühren bei 60°C eine Mischung aus 276,73 g Polyoxyethylen-polyoxypropylen-Diol 1900 (Pluronic^R L 35, Wyandotte Chemicals Corp.) und 29,77 g Polyoxyethylen-(40)-glycerol-tri-hydroxystearat (Cremophor^R RH 40, BASF) her und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 9

Man rührt bei 60°C 90,00 g Ibuprofen portionsweise in 306,00 g Polyoxyethylen-polyoxypropylen-Diol 1900 (Pluronic^R L 35, Wyandotte Chemicals Corp.) ein. Die Lösung wird auf Raumtemperatur abgekühlt und in Weichgelatinekapseln geeigneter Größe eingearbeitet. 0,880 g Lösung entahlten 200 mg Ibuprofen.

Beispiel 10

Man rührt bei 60°C 90,00 g Ibuprofen portionsweise in 306,00 g Polyoxyethylen-polyoxypropylen-Diol 1500 (Pluronic^R L 42, Wyandotte Chemicals Corp.) ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 11

Bie 60°C werden 90,00 g Ibuprofen portionsweise in 306,00 g Polyoxyethylen-polyoxypropylen-Diol 2000 (Pluronic^R L 44, Wyandotte Chemicals Corp.) eingerührt. Die Lösung wird auf Raumtemperatur abgekühlt und in Weichgelatinekapseln geeigneter Größe eingearbeitet. 0,880 g Lösung enthalten 200 mg Ibuprofen.

Beispiel 12

Man stellt unter Rühren bei 60 bis 70°C eine Mischung aus 149,94 g Polyethylenglykol 400, 149,94 g Polyoxyethylen-polyoxypropylen-Diol 1900 (Pluronic^R L 35, Wyandotte Chemicals Corp.) und 6,12 g 1,2-Propylenglykol her und rührt portionsweise 90,00 g Ibuprofen ein. Die Lösung wird auf Raumtemperatur abgekühlt und in Weichgelatinekapseln geeigneter Größe eingearbeitzt. 0,880 g Lösung enthalten 200 mg Ibuprofen.

Beispiel 13

Man stellt unter Rühren bei 60 bis 70°C eine Mischung aus 270,62 g Polyethylenglykol 400, 29,26 g Polyoxyethylen-(20)-stearylalkohol (BRIJ^R 78, Atlas Chemical Industries) und 6,12 g 1,2-Propylenglykol her, kühlt auf 50°C und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 14

Man stellt unter Rühren bie 60 bis 70°C eine Mischung aus 270,62 g Polyoxyethylen-polyoxypropylen-Diol 1900 (Pluronic^R L 35, Wyandotte Chemicals Corp.), 29,26 g Polyoxyethylen-(30)-stearat (MYRJ^R 51, Atlas Chemical Industries) und 6,12 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 15

Man stellt unter Rühren bei 60 bis 70°C eine Mischung aus 270,62 g Polyoxyethylen-polyoxypropylen-Diol 1900 (Pluronic$^R$ L 35, Wyandotte Chemicals Corp.), 29,26 g Polyoxyethylen-(20)-stearylalkohol (BRIJ$^H$ 78, Atlas Chemical Industries) und 6,12 g 1,2-Propylenglykol her, kühlt auf 50°C und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 16

Man stellt unter Rühren bei 60 bis 70°C eine Mischung aus 270,62 g Polyethylenglykol 400, 29,26 g Polyoxyethylen-(60)-glycerol-tri-hydroxystearat (Cremophor$^R$ RH 60, BASF) und 6,12 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 17

Man stellt unter Rühren bei 60 bis 70°C eine Mischung aus 270,62 g Polyoxyethylen-polyoxypropylen-Diol 1900 (Pluronic$^R$ L 35, Wyandotte Chemicals Corp.), 29,26 g Polyoxyethylen-(60)-glycerol-tri-hydroxystearat (Cremophor$^R$ RH 60, BASF) und 6,12 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 18

Man stellt unter Rühren bei 60 bis 70°C eine Mischung aus 270,62 g Polyoxyethylen-polyoxypropylen-Diol 1500 (Pluronic$^R$ L 42, Wyandotte Chemicals Corp.), 29,26 g Polyoxyethylen-(20)-sorbitan-monostearat. (Tween$^R$ 60, Atlas Chemical Industries) und 6,12 g 1,2-Propylenglykol her, kühlt auf 50°C ab und rührt portionsweise 90,00 g Ibuprofen ein. Die Lösung wird auf Raumtemperatur abgekühlt und in Weichgelatinekapseln geeigneter Größe eingearbeitet. 0,880 g Lösung enthalten 200 mg Ibuprofen.

Beispiel 19

Man stellt unter Rühren bei 60 bis 70°C eine Mischung aus 270,62 g Polyoxyethylen-polyoxypropylen-Diol 1900 (Pluronic$^R$ L 35, Wyandotte Chemicals Corp.), 29,26 g Polyoxyethylen-(40)-glycerol-tri-hydroxystearat (Cremophor$^R$ RH 40, BASF) und 6,12 g 1,2-Propylenglykol her, kühlt auf 50°C ab und rührt portionsweise 90,00 g Ibuprofen ein. In dieser Lösung werden bei 15 bis 20°C weitere 90,00 g feinverteiltes Ibuprofen suspendiert. Die so erhaltene Suspension wird in Weichgelatinekapseln geeigneter Größe eingearbeitet. 1,080 g Suspension enthalten 400 mg Ibuprofen.

Beispiel 20

Man stellt unter Rühren bei 60 bis 70°C eine Mischung aus 202,97 g Polyoxyethylen-polyoxypropylen-Diol 1900 (Pluronic$^R$ L 35, Wyandotte Chemicals Corp.), 21,95 g Polyoxyethylen-(40)-glycerol-tri-hydroxystearat (Cremophor$^R$ RH 40, BASF) und 4,59 g 1,2-Propylenglykol her, kühlt auf 50°C ab und rührt portionsweise 67,50 g Ibuprofen ein. In dieser Lösung werden bei 15 bis 20°C weitere 112,50 g feinverteiltes Ibuprofen suspendiert. Die so erhaltene Suspension wird in Weichgelatinekapseln geeigneter Größe eingearbeitet, 0,910 g Suspension enthalten 400 mg Ibuprofen.

Die Freisetzung des Ibuprofens aus den Weichgelatinekapseln wurde mittels Dissolution-Test nach USP XX, 5th Suppl., in 4,0 l künstlichem Magensaft entsprechend USP XX ohne Pepsin bei 37°C geprüft. Die Konzentration des Ibuprofens wurde UV-spektrometrisch bei 231 nm bestimmt. Zum Vergleich wurden ein handelsübliches Dragée und die Lösungen A und B herangezogen. 0.880 g der Lösung A enthält 200 mg Ibuprofen, 666,4 mg Polyethylenglykol 400 und 13,6 mg 1,2-Propylenglykol. 0,880 g der Lösung B enthält 200 mg Ibuprofen und 680,0 mg Polyethylenglykol 400.

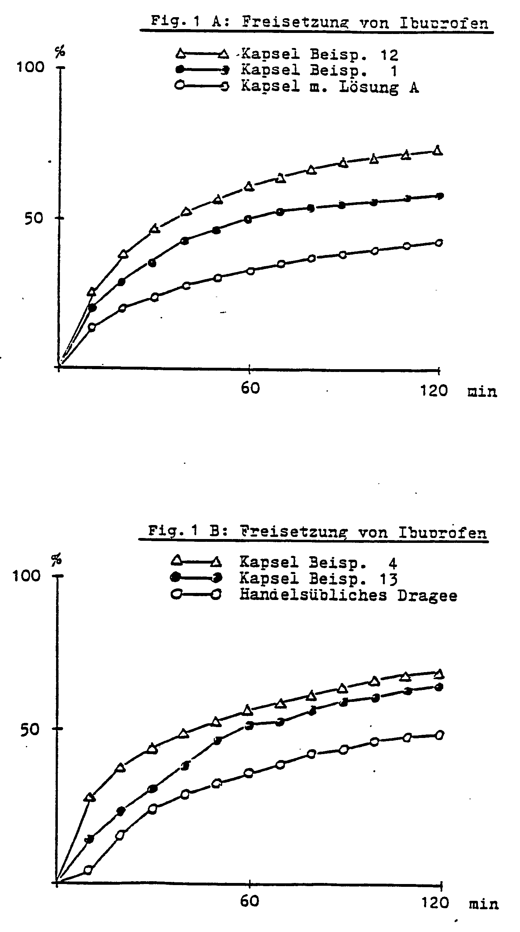

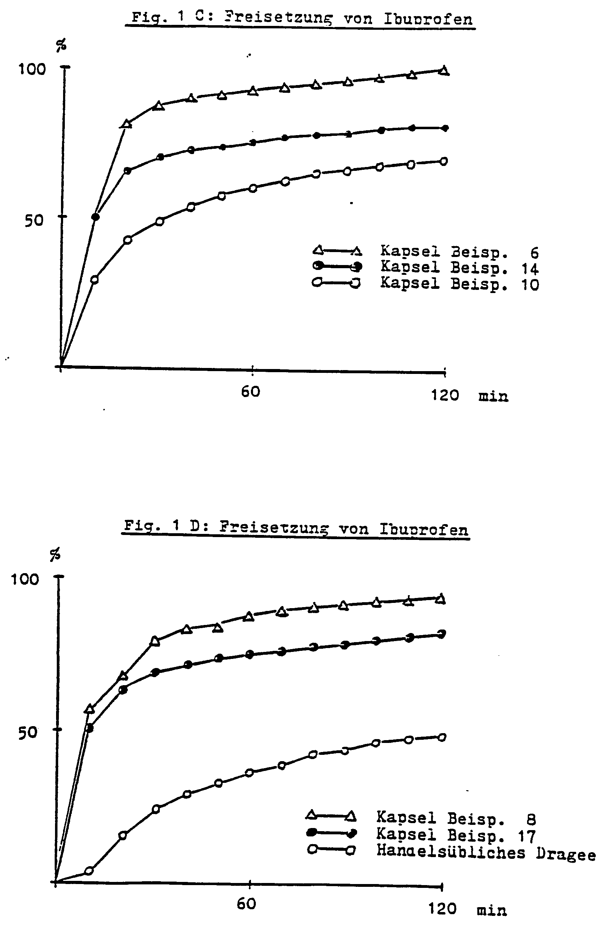

In Tab. 1 sind die Freisetzungsraten der erfindungsgemäßen Weichgelatinekapseln und der Vergleichspräparate zusammengestellt. Die Figuren 1A bis 1D veranschaulichen zusätzlich den Verlauf der Freisetzung einiger repräsentativer Darreichungsformen. Daraus ist einwandfrei zu erkenne, daß das Ibuprofen aus den erfindungsgemäßen Weichgelatinekapseln wesentlich schneller freigesetzt wird als aus dem handelsüblichen Dragée und aus Kapseln, die die Vergleichslösung enthalten. Außerdem kommt die Freisetzung des Ibuprofens aus den Vergleichspräparaten nach 2 Stunden bei weniger als 48% zum Stillstand während sie bei den erfindungsgemäßen Präparaten Werte bis 100% erreicht.

TABELLE 1

| | Freisetzung von Ibuprofen in % nach | | | | |
| --- | --- | --- | --- | --- | --- |
| | 10 | 20 | min. 30 | 60 | 120 |
| Handelsübliches Dragée | 3,7 | 15,0 | 23,7 | 36,2 | 48,7 |
| Kapsel mit Lösung A | 13,7 | 20,0 | 23,7 | 32,5 | 42,5 |
| Kapsel mit Lösung B | 16,2 | 22,5 | 27,5 | 37,5 | 47,5 |
| Kapsel Beispiel 1 | 20,0 | 28,7 | 35,0 | 50,0 | 58,7 |
| Kapsel Beispiel 4 | 27,5 | 37,5 | 43,7 | 56,2 | 68,7 |
| Kapsel Beispiel 5 | 20,0 | 31,2 | 40,0 | 55,9 | 68,2 |
| Kapsel Beispiel 6 | 50,0 | 81,2 | 87,5 | 92,5 | 100,0 |
| Kapsel Beispiel 8 | 56,2 | 67,0 | 78,7 | 87,5 | 94,7 |
| Kapsel Beispiel 9 | 27,5 | 41,2 | 48,7 | 61,2 | 72,5 |
| Kapsel Beispiel 10 | 28,7 | 42,5 | 48,7 | 60,0 | 70,0 |
| Kapsel Beispiel 11 | 21,2 | 37,5 | 46,2 | 61,2 | 73,0 |
| Kapsel Beispiel 12 | 28,7 | 42,5 | 48,7 | 60,0 | 72,9 |
| Kapsel Beispiel 13 | 21,2 | 37,5 | 46,2 | 61,2 | 64,1 |
| Kapsel Beispiel 14 | 50,0 | 65,0 | 70,0 | 75,0 | 81,0 |
| Kapsel Beispiel 15 | 40,0 | 56,2 | 63,7 | 75,0 | 83,7 |
| Kapsel Beispiel 16 | 13,7 | 22,5 | 30,0 | 51,2 | 60,0 |
| Kapsel Beispiel 17 | 50,0 | 62,5 | 68,7 | 75,0 | 82,5 |
| Kapsel Beispiel 18 | 58,7 | 67,5 | 70,0 | 75,0 | 78,2 |

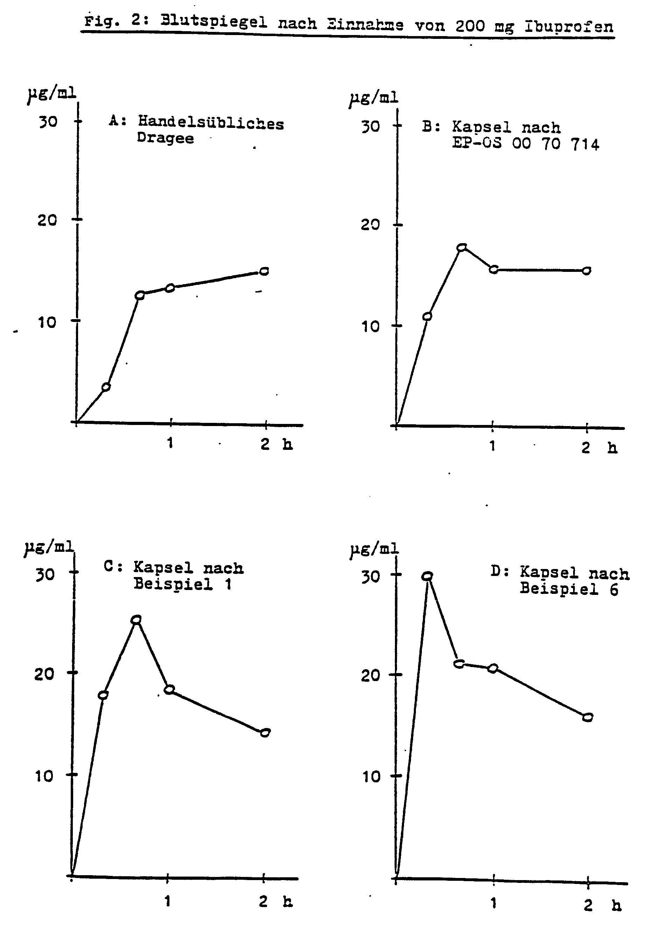

Die Bioverfügbarkeit des Ibuprofens aus den erfindungsgemäßen Kapseln, einem handelsüblichen Dragée und einer Kapsel nach EP-OS 0 070 714 (Beispiel 2) wurde an 4 Probanden geprüft. Dazu wurde den Probanden einmalig eine Kapsel bzw. ein Dragée mit jeweils 200 mg Ibuprofen verabreicht, nach bestimmten Zeiten Blut entnommen und der Ibuprofen-Gehalt bestimmt. Die Mittelwerte der Bestimmungen sind in den Figuren 2 A bis D dargestellt. Unter der Bioverfügbarkeit eines Arzneistoffes aus einer Zubereitung versteht man die Geschwindigkeit und das Ausmaß, mit denen dieser in die Blutbann gelangt (E. Mutschler, Arzneimittel-Wirkungen, Wissenschaftliche Verlagsgesellschaft mbH, Stuttgart 1981). Nach F. H. Dost (Grundlagen der Pharmakokinetik, Georg Thieme Verlag, Stuttgart 1968) entspricht die Fläche, welche die Blutspiegelkurve mit der Zeitachse umschließt (AUC=area under the curve) der Substanzmenge im Organismus. Daraus ergibt sich die Möglichkeit, die Bioverfügbarkeit einer Substanz aus verschiedenen Darreichungsformen zu vergleichen. Aus den in Figuren 2 A bis D dargestellten Blutspiegelverläufen wurden die AUC-Werte berechnet und die relative Bioverfügarkeit des Ibuprofens ermittelt (Tabelle 2).

### TABELLE 2

|  | AUC-Fläche mm² | Relative Bioverfügbarkeit | |
|---|---|---|---|
| Handelsübliches dragée | 1466,9 | 1 | 0,79 |
| Kapsel nach EP-OS 00 70 714 | 1866,0 | 1,27 | 1 |
| Kapsel nach Beispiel 1 | 2270,9 | 1,55 | 1,22 |
| Kapsel nach Beispiel 6 | 2605,0 | 1,78 | 1,40 |

Tabelle 2 zeigt, daß mit den erfindungsgemäßen Weichgelatinekapseln die Bioverfügbarkeit des Ibuprofens gegenüber dem handelsüblichen Dragée bzw. einem vorbeschriebenen Kapselpräparat µm 55 und 78% bzw. um 22 und 40% verbessert wurde. Darüberhinaus ist, wie aus dem Vergleich des Anstiegs der Blutspiegelkurven der Figuren 2 A bis D ersichtlich ist, bei den erfindungsgemäßen Kapseln eine schnellere Anflutung des Wirkstoffes als bei den vorbekannten Präparaten gegeben. Mit der schnelleren und höhren Freisetzung und Bioverfügbarkeit von Ibuprofen aus den erfindungsgemäßen Kapseln steht somit ein besser und schneller wirksames Ibuprofen enthaltendes Arzneimittel als bisher zur Verfügung.

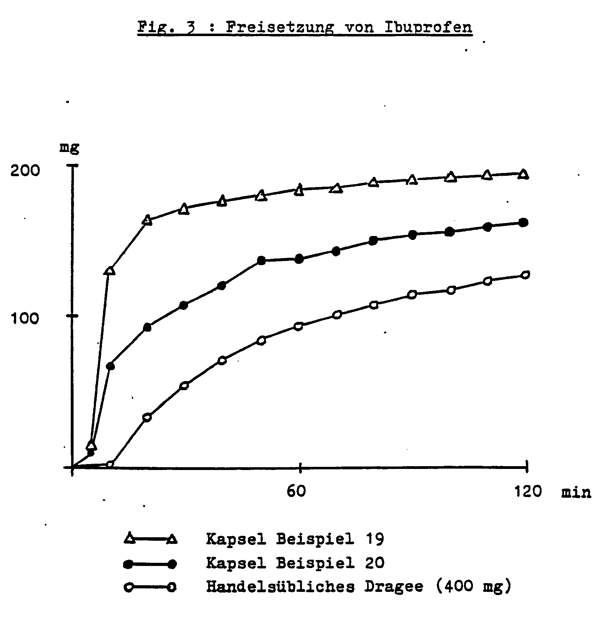

Tabelle 3 zeigt die Werte der Beispiele 19 und 20 in mg Ibuprofen im Vergleich zu den handelsüblichen Dragees, da bei der Messung der Freisetzung das Volumen des Mediums aus technischen Gründen nicht erhöht werden kann und bei höhren Wirkstoffdosen rasch Sättigung erreicht wird. Aber auch hierbei zeigt sich ein deutlich besserer Effekt als bei den handelsüblichen Dragees. In Figur 3 sind die gefundenen Werte noch einmal graphisch zusammengestellt.

### TABELLE 3

|  | Freisetzung von Ibuprofen in mg nach . . . min. | | | | |
|---|---|---|---|---|---|
|  | 10 | 20 | 30 | 60 | 120 |
| Handelsübliches Dragee (400 mg) | 1,8 | 32,8 | 54,7 | 93,1 | 125,2 |
| Kapsel Beispiel 19 | 128,1 | 163,3 | 171,5 | 183,9 | 195,8 |
| Kapsel Beispiel 20 | 67,0 | 93,0 | 108,4 | 137,8 | 162,5 |

**Patentansprüche**

1. Ibuprofen enthaltende Weichgelatinekapseln, dadurch gekennzeichnet, daß sie eine Lösung von 15 bis 30 Gewichtsteilen Ibuprofen in 70 bis 85 Gewichtsteilen Polyoxyethylen-polyoxypropylen-Diol oder in einem Gemisch aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids enthalten.

2. Weichgelatinekapseln gemäß Anspruch 1, dadurch gekennzeichnet, daß sie zusätzlich bis zu 3 Gewichtsteile 1,2-Propylenglykol enthalten.

3. Weichgelatinekapseln gemäß Anspruch 1 oder 2, daß sie zusätzlich zum gelösten Ibuprofen bis zu 40 Gewichtsteile suspendiertes Ibuprofen enthalten.

4. Weichgelatinekapseln gemäß einem der Ansprüche 1 bis 3, daß sie als Tensid Polyoxyethylen-glycerol-tri-hydroxystearat, Polyoxyethylen-($C_{12-18}$)-fettalkoholether, Polyoxyethylen-stearat, Polyoxyethylen-sorbitan-mono($C_{12-18}$)-fettsäureester, Polyoxyethylen-polyoxypropylen-Diol oder Gemische derselben enthalten.

5. Verfahren zur Herstellung von Ibuprofen enthaltenden Weichgelatinekapseln, dadurch gekennzeichnet, daß 15 bis 30 Gewichtsteile Ibuprofen bei Temperaturen zwischen 45 und 65°C in 70 bis 85 Gewichtsteilen Polyoxyethylen-polyoxypropylen-Diol oder in einem Gemisch aus 30 bis 76 Gewichtsteilen

# EP 0 178 436 B1

Polyoxyethylen-polyoxypropylen-Diol oder Polyethylenglykol oder Polypropylenglykol und 7 bis 40 Gewichtsteilen eines physiologisch verträglichen Tensids gelöst, auf Raumtemperatur abgekühlt und in an sich bekannter Weise in Weichgelatinekapseln eingearbeitet werden.

6. Verfahren gemäß Anspruch 5, dadurch gekennzeichnet, daß in den Lösungsmitteln bis zu 3 Gewichtsteilen 1,2-Propylenglykol zugesetzt werden.

7. Verfahren gemäß Anspruch 5 oder 6, dadurch gekennzeichnet, daß in der Lösung nach dem Abkühlen zusätzlich bis zu 40 Gewichtsteilen Ibuprofen suspendiert werden.

8. Verfahren gemäß einem der Ansprüche 5 bis 7, dadurch gekennzeichnet, daß als Tensid Polyoxyethylen-glycerol-tri-hydroxystearat, Polyoxyethylen-(C$_{12-18}$)-fettalkoholether, Polyoxyethylen-stearat, Polyoxyethylen-sorbitan-mono-(C$_{12-18}$)-fettsäureester, Polyoxyethylen-polyoxypropylen-Diol oder Gemische derselben verwendet werden.

9. Verwendung von Lösungen enthaltend 15 bis 30 Gewichtsteile Ibuprofen in 70 bis 85 Gewichtsteilen Polyoxyethylen-Polyoxypropylen-Diol oder in einem Gemisch aus 30 bis 76 Gewichtsteilen Polyoxyethylen-polyoxypropylen-Diol oder Polyethylenglykol oder Polypropylenglykol und 7 bis 40 Gewichtsteilen eines physiologisch verträglichen Tensids zur Herstellung von Weichgelatinekapseln.

## Revendications

1. Capsules de gélatine molle contenant de l'ibuprofen, caractérisées en ce qu'elles contiennent une solution de 15 à 30 parties en poids d'ibuprofen dans 70 à 85 parties en poids de polyoxyéthylène-polyoxypropylène-diol ou dans un mélange de 30 à 76 parties en poids de polyoxyéthylène-polyoxypropylène-diol ou de polyéthylèneglycol ou de polypropylène-glycol et de 7 à 40 parties en poids d'un produit tensioactif toléré par l'organisme.

2. Capsules de gélatine molle selon la revendication 1, caractérisées en ce qu'elles contiennent en plus jusqu'à 3 parties en poids de propylène-glycol-1,2.

3. Capsules de gélatine molle selon la revendication 1 ou 2, caractérisées en ce qu'elles contiennent, en plus de l'ibuprofen dissous, jusqu'à 40 parties en poids d'ibuprofen en suspension.

4. Capsules de gélatine molle selon l'une des revendications 1 à 3, caractérisées en ce qu'elles contiennent comme produit tensioactif du trihydroxystéarate de polyoxyéthylène-glycérol, d'un éther de polyoxyéthylène-alcool gras (C$_{12}$—C$_{18}$), du stéarate de polyoxyéthylène, d'un ester de polyoxyéthylène-sorbitan-mono acide gras (C$_{12}$—C$_{18}$), de polyoxyéthylène-polyoxypropylène-diol ou de mélanges de ceux-ci.

5. Procédé de préparation de capsules de gélatine molle contenant l'ibuprofen, caractérisé en ce que 15 à 30 parties en poids d'ibuprofen sont dissoutes à des températures comprises entre 45 et 65°C dans 70 à 85 parties en poids de polyoxyéthylène-polyoxypropylène-diol ou dans un mélange de 30 à 76 parties en poids de polyoxyéthylène-polyoxypropylène-diol ou de polyéthylène-glycol ou de polypropylène-glycol et de 7 à 40 parties en poids d'un produit tensio-actif toléré par l'organisme, puis sont refroidies à la température ambiante et transformées en capsules de gélatine molle de manière en soi connue.

6. Procédé selon la revendication 5, caractérisé en ce qu'il est ajouté dans les solvants jusqu'à 3 parties de poids de polypropylène-glycol-1,2.

7. Procédé selon la revendication 5 ou 6, caractérisé en ce qu'il est mis en suspension en plus dans la solution après refroidissement jusqu'à 40 parties en poids d'ibuprofen.

8. Procédé selon l'une des revendications 5 à 7, caractérisé en ce qu'il est utilisé comme produit tensioactif du trihydroxystéarate de polyoxyéthylèneglycérol d'un éther de polyoxyéthylène-alcool gras (C$_{12}$—C$_{18}$), du stéarate de polyoxyéthylène, d'un ester de polyoxyéthylène-sorbitan-mono acide gras (C$_{12}$—C$_{18}$), ou de mélanges de ceux-ci.

9. Utilisation de solutions contenant 15 à 30 parties en poids d'ibuprofen dans 70 à 85 parties en poids de polyoxyéthylène-polyoxypropylène-diol ou dans un mélange comprenant 30 à 76 parties en poids de polyoxyéthylène-polyoxypropylène-diol ou de polyéthylène-glycol ou de polypropylène-glycol et de 7 à 40 parties en poids d'un produit tensioactif toléré par l'organisme pour la fabrication de capsules de gélatine molle.

## Claims

1. Soft gelatin capsules containing ibuprofen, characterized in that they contain a solution of from 15 to 30 parts by weight of ibuprofen in from 70 to 85 parts by weight of polyoxyethylene-polyoxypropylene-diol or in a mixture comprising from 30 to 76 parts by weight of polyoxyethylene-polyoxypropylene-diol or polyethyleneglycol or polypropyleneglycol and from 7 to 40 parts by weight of a physiologically compatible surfactant.

2. Soft gelatin capsules according to Claim 1, characterized in that they additionally contain up to 3 parts by weight of 1,2-propyleneglycol.

3. Soft gelatin capsules according to Claims 1 or 2, characterized in that they contain up to 40 parts by weight of suspended ibuprofen in addition to the dissolved ibuprofen.

4. Soft gelatin capsules according to any of Claims 1 to 3, characterized in that they contain

8

polyoxyethylene-glycerol-tri-hydroxystearate, polyoxyethylene-(C$_{12-18}$)-fatty alcohol ether, polyoxyethylene-stearate, polyoxyethylene-sorbitan-mono(C$_{12-18}$)-fatty acid ester, polyoxyethylene-polyoxypropylene-diol or mixtures thereof as a surfactant.

5. A process for the preparation of soft gelatin capsules containing ibuprofen, characterized in that from 15 to 30 parts by weight of ibuprofen are dissolved in 70 to 85 parts by weight of polyoxyethylene-polyoxypropylene-diol or in a mixture comprising 30 to 76 parts by weight of polyoxyethylene-polyoxypropylene-diol or polyethyleneglycol or polypropyleneglycol and 7 to 40 parts by weight of a physiologically compatible surfactant at temperatures of between 45°C and 65°C, allowed to cool to room temperature and are incorporated in soft gelatin capsules in a *per se* known manner.

6. The process according to Claim 5, characterized in that up to 3 parts by weight of 1,2-propyleneglycol are added in the solvents.

7. The process according to Claims 5 or 6, characterized in that in addition up to 40 parts by weight of ibuprofen are suspended in the solution after cooling.

8. The process according to any of Claims 5 to 7, characterized in that polyoxyethylene-glycerol-trihydroxystearate, polyoxyethylene-(C$_{12-18}$)-fatty alcohol ether, polyoxyethylene-stearate, polyoxyethylene-sorbitan-mono-(C$_{12-18}$)-fatty acid ester, polyoxyethylene-polyoxypropylene-diol or mixtures thereof are used as surfactant.

9. Use of solutions containing from 15 to 30 parts by weight of ibuprofen in from 70 to 85 parts by weight of polyoxyethylene-polyoxypropylene-diol or in a mixture comprising 30 to 76 parts by weight of polyoxyethylene-polyoxypropylene-diol or polyethyleneglycol or polypropyleneglycol and 7 to 40 parts by weight of a physiologically compatible surfactant for the preparation of soft gelatin capsules.

Fig. 1 A: Freisetzung von Ibuprofen

△———△ Kapsel Beisp. 12
●———● Kapsel Beisp. 1
○———○ Kapsel m. Lösung A

Fig. 1 B: Freisetzung von Ibuprofen

△———△ Kapsel Beisp. 4
●———● Kapsel Beisp. 13
○———○ Handelsübliches Dragee

Fig. 1 C: Freisetzung von Ibuprofen

Kapsel Beisp. 6
Kapsel Beisp. 14
Kapsel Beisp. 10

Fig. 1 D: Freisetzung von Ibuprofen

Kapsel Beisp. 8
Kapsel Beisp. 17
Handelsübliches Dragee

Fig. 2: Blutspiegel nach Einnahme von 200 mg Ibuprofen

A: Handelsübliches Dragee

B: Kapsel nach EP-OS 00 70 714

C: Kapsel nach Beispiel 1

D: Kapsel nach Beispiel 6

EP 0 178 436 B1

3

## Fig. 3 : Freisetzung von Ibuprofen

△——△ Kapsel Beispiel 19
●——● Kapsel Beispiel 20
○——○ Handelsübliches Dragee (400 mg)